# EUROPEAN PATENT APPLICATION

(11) **EP 2 474 332 A1**
(43) Date of publication of application: **11.07.2012**
(21) Application number: 11150413.0
(22) Date of filing: 07.01.2011
(51) Int. Cl.: A61M 5/24

(54) **Cartridge holder for an injection device**

(71) Applicant: Sanofi-Aventis Deutschland GmbH, 65929 Frankfurt (DE)
(72) Inventor: The designation of the inventor has not yet been filed

(57) **Abstract**

A cartridge holder (1) suitable for an injection device is proposed. The injection device is adapted to administer a dose of a medicament to a patient. The cartridge holder (1) is adapted to receive a medical cartridge (2) containing the dose of the medicament. The cartridge holder (1) comprises an annular collar (1.2) with a first set (S1) of first detents (1.2.1) arranged around a circumference of the annular collar (1.2). Each first detent (1.2.1) projects from an inner surface of the annular collar (1.2) in a radial inward direction. The first detent (1.2.1) is adapted to engage a corresponding second detent (2.2) of the medical cartridge (2) to mount the medical cartridge (2) to the cartridge holder (1). The first detent (1.2.1) is resiliently arranged and/or supported so as to provide a unidirectional torque limiter that restricts a maximal torque between the cartridge holder (1) and the medical cartridge (2) in a first rotational direction (R1).

## Description

### Technical Field

The invention relates to cartridge holders adapted to mount a medical cartridge to an injection device. More particularly the cartridge holder is adapted to mount the medical cartridge to an auto-injector, a pen injector or a safety syringe.

### Background of the Invention

Injection devices known in the state of the art comprise cartridge holders or syringe retainers that are adapted to mount a medical cartridge or a syringe that may be prefilled with a dose of the medicament within the injection device. In the case of needleless medical cartridges, it is common to attach a needle hub carrying an injection needle to the medical cartridge. The injection needle may in particular be arranged as a double-ended needle that pierces a septum of the medical cartridge when the needle hub is attached thereunto.

### Summary of the Invention

It is an object of the present invention to provide an improved cartridge holder ensures re-usability and/or operability of an injection device.

The object is achieved by a cartridge holder according to claim 1 and by an assembly comprising a cartridge holder and a medical cartridge according to claim 2.

Preferred embodiments of the invention are given in the dependent claims.

In the context of this specification, the terms distal and proximal are defined from the point of view of a person performing an injection. Consequently, a distal direction refers to a direction pointing towards the body of a patient receiving an injection and a distal end defines an end of an element that is directed towards the body of the patient. Respectively, the proximal end of an element or the proximal direction is directed away from the body of the patient receiving the injection and opposite to the distal end or distal direction.

According to the invention, a cartridge holder for an injection device capable of administering a dose of a medicament is adapted to receive a medical cartridge containing the dose of the medicament. The cartridge holder comprises an annular collar with a first set of first detents arranged around a circumference of the annular collar. Each first detent projects from an inner surface of the annular collar in a radial inward direction. The first detent is adapted to engage a corresponding second detent of the medical cartridge to mount the medical cartridge to the cartridge holder. The first detent is resiliently arranged and/or supported so as to provide a unidirectional torque limiter that restricts a maximal torque between the cartridge holder and the medical cartridge in a first rotational direction.

The torque limiter is generally arranged to prevent a potential jamming when the injection device comprising is assembled or during use of the injection device. The medical cartridge may particularly be designed as a needleless ampoule with a screw thread that receives a needle hub carrying a needle. During assembly of the injection device, the medical cartridge may be retained in the cartridge holder while the needle hub is screwed onto a distal end of the medical cartridge. If the torque exerted upon the medical cartridge to affix the needle hub thereto exceeds a critical value, the medical cartridge might get stuck and jammed within the cartridge holder rendering the device inoperable or non-reusable. The cartridge holder is arranged to limit the torque that may occur between the medical cartridge and the cartridge holder to a maximal value by means similar to a slipping clutch. The torque is only limited in the first rotational direction, so that the needle hub may easily be unscrewed after the injection is completed by a rotation in an opposite, second rotational direction. The first detents are resiliently arranged or resiliently supported, so that the first detents may overcome the second detents when the torque applied in the first rotational direction exceeds the maximal value.

However, the above mentioned example of a medical cartridge that receives a needle hub is only one possible example to illustrate the usefulness of the underlying invention. Nowadays injection device comprise sophisticated mechanisms that may exert a torque on various parts of the injection device to accomplish different tasks, like for example the insertion of an injection needle into the skin of a patient, the withdrawal of the injection needle, the shielding of the injection needle before or after the injection or the advancing of a plunger. In particular when parts of the injection device are driven by an electric motor, the torque limiter provided by the first set of first detents may avoid an overload that damages the injection device. Generally, the medical cartridge or the cartridge holder may be adapted to be mounted to an element by rotation. The element may carry, like the above mentioned needle hub, another member, in particular the injection needle, a plunger or a shielding device providing needle safety for the injection needle.

According to a possible embodiment of the invention, the first detents of the first set arranged around the circumference of the annular collar are regularly spaced apart from each other. The arrangement of the first detents at regular intervals ensures that at least one of the first detents engages at least one of the second detents to provide the unidirectional torque limiter.

According to another possible embodiment of the invention, the first set of first detents is adapted to engage the medical cartridge so as to limit an axial displacement of the medical cartridge with respect to the cartridge holder. The number of first detents in the first set may differ from the number of the second detents in the second set. At any time, there is a subset of first detents that are not engaged and thus not deflected. This subset of first detents abuts the medical cartridge so as to retain the medical cartridge and the cartridge holder in a fixed axial arrangement with respect to each other.

According to yet another possible embodiment of the invention, the annular collar is integrally formed with the cartridge holder. The annular collar and the cartridge holder are arranged as one piece to reduce production costs.

Preferably, the axial length of the cartridge holder substantially corresponds to an axial dimension of the medical cartridge. The medical cartridge is inserted into the cartridge holder and safely retained therein so as to provide a reliable mounting means mounting the medical cartridge to the injection device.

Each of the first detents may have a first surface and a second surface parallel to the first surface, wherein the first surface is inclined with respect to an axis of the cartridge holder. The inclination of the first and the second surfaces with respect to the axis introduces a preferred rotational orientation so as to provide the unidirectional torque limiter. The first detent is arranged be deflected only in one rotational direction, whereas a deflection of the first detent in the opposite rotational direction is prevented.

According to yet another possible embodiment of the invention, each of the first detents comprises a free end separated from the annular collar by an undercut. The free end is resiliently supported and is deflected if the torque occurring between the cartridge holder and the medical cartridge reaches the maximal value, so that the second detents may overcome the first detent.

The annular collar may be arranged at a distal end of the cartridge holder or, alternatively, at a proximal end of the cartridge holder.

Although the deflectable first detents are arranged on the inner surface of the cartridge holder and the second detents are arranged on the outer surface of the medical cartridge in the embodiment described herein below, equivalent arrangements are within the scope of the present invention. In particular, the deflectable first detents may be arranged on the medical cartridge and the second detents may project inwards from the inner surface of the cartridge holder.

An assembly of a medical cartridge and a cartridge holder as described herein before is proposed, wherein the medical cartridge is retained within the cartridge holder. The medical cartridge comprises a second set of second detents arranged around a circumference of the medical cartridge. The second detents project in a radial outward direction to engage the first detents and at least one second detent engages at least one first detent to provide the unidirectional torque limiter. The assembly of the cartridge holder and medical cartridge combines the before mentioned advantages and in particular prevents the medical cartridge from getting jammed within the cartridge holder. According to a possible embodiment of the invention, the second detent comprises a triangular shape with an inclined side arranged to abut on second surface of the first detent. The inclined side of the second detent bears against the second surface to allow for a deflection of the first detent in the first rotational direction when the applied torque reaches the maximal value. The torque that may occur between the cartridge holder and the medical cartridge in the first rotational direction is thus restricted to the maximal value.

According to another possible embodiment of the invention, the second detents of the second set are arranged around the circumference of the medical cartridge and are regularly spaced apart from each other. The second set may comprise a lower number of second detents compared to the number of first detents in the first set. Each second detent is retained between to adjacent first detents, so that the second detent engages one first detent when a torque is applied in the first or the second rotational direction.

Preferably, the medical cartridge comprises an annular flange arranged to abut against at least one of the first detents to limit an axial translation of the medical cartridge with respect to the cartridge holder. In particular the subset of first detents that are not deflected may bear against the annular flange to prevent the axial translation of the medical cartridge in the distal direction. The cartridge holder hence provides a suitable mounting means for releasably mounting the medical cartridge to the injection device.

Preferably, the medical cartridge comprises a screw thread adapted to be connected to a correspondingly threaded element. An orientation of rotation of the screw thread corresponds to the first rotational direction limited by the torque limiter of the cartridge holder so as to allow for an attachment of the element to the medical cartridge by a rotation directed in the first rotational direction. The torque limiter prevents that the screw thread is fastened too tight so that the element may be easily detached from the medical cartridge retained within the cartridge holder.

According to yet another possible embodiment of the invention, the screw thread is arranged at a distal end section of the medical cartridge and protrudes from the cartridge holder in a distal direction so as to allow the element to be attached to a distal end section of the medical cartridge.

Alternatively, the element may be attachable to the medical cartridge by a bayonet type of coupling.

The element that is attached to cartridge may comprise a needle hub with an injection needle attached thereto. The injection needle may in particular be arranged as a double ended needle with a sharpened distal and a sharpened proximal tip. A septum of the medical cartridge may be pierced by the proximal tip of the injection needle when the needle hub is attached to the medical cartridge to establish a fluid communication with the medical cartridge, so that the dose of the medicament contained in the medical cartridge may be expelled through the injection needle and disposed beneath the skin of the patient receiving the injection.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows the cartridge holder in an isometric view;
- Figure 2: shows details of an annular collar formed to the cartridge holder in an isometric view;
- Figure 3: shows a section of the annular collar formed to the distal end of the cartridge holder in an isometric view;
- Figure 4: shows an isometric top view of the annular collar;
- Figure 5: isometric view of a substantially cylindrical medical cartridge;
- Figure 6: shows a section of the cartridge holder with a medical cartridge retained therein in an isometric view.

Corresponding parts are marked with the same reference symbols in all figures.

### Detailed Description of Preferred Embodiments

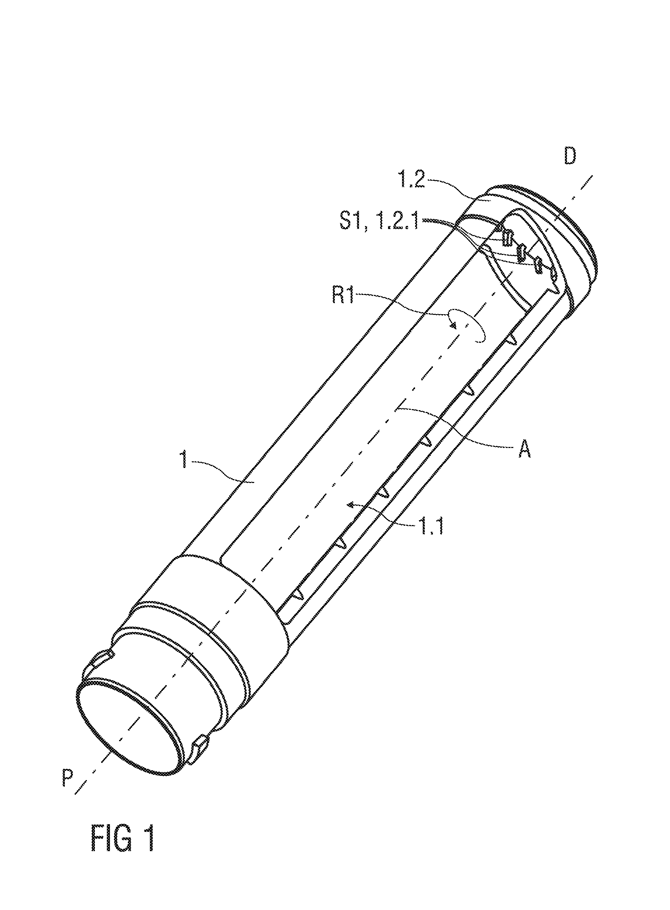

Figure 1 shows a cartridge holder 1 adapted to receive a medical cartridge 2. The cartridge holder 1 mounts the medical cartridge 2 to an injection device (not illustrated) that may be used to administer a dose of a medicament contained in the medical cartridge 2 to a patient. In particular, the injection device may be arranged as a safety syringe, an auto-injector, a pen injector or the like.

The cartridge holder 1 has a substantially cylindrical shape with a longitudinal axis A and comprises a distal and a proximal end. Two windows 1.1 are formed to the lateral walls of the cartridge holder 1 so as to retain the medical cartridge 2 retained therein in a manner that allows the user to see a preferable transparent barrel 2.1 of the medical cartridge 2 containing the dose of the medicament. An axial length of the cartridge holder 1 extending parallel to the axis A substantially corresponds to an axial dimension of the medical cartridge 2.

An annular collar 1.2 is integrally formed to the distal end of the cartridge holder 1. The annular collar 1.2 comprises a first set S1 of radial inwardly projecting first detents 1.2.1 formed to an inner surface of the annular collar 1.2. The first detents 1.2.1 are arranged around a circumference of the annular collar 1.2 in regular intervals.

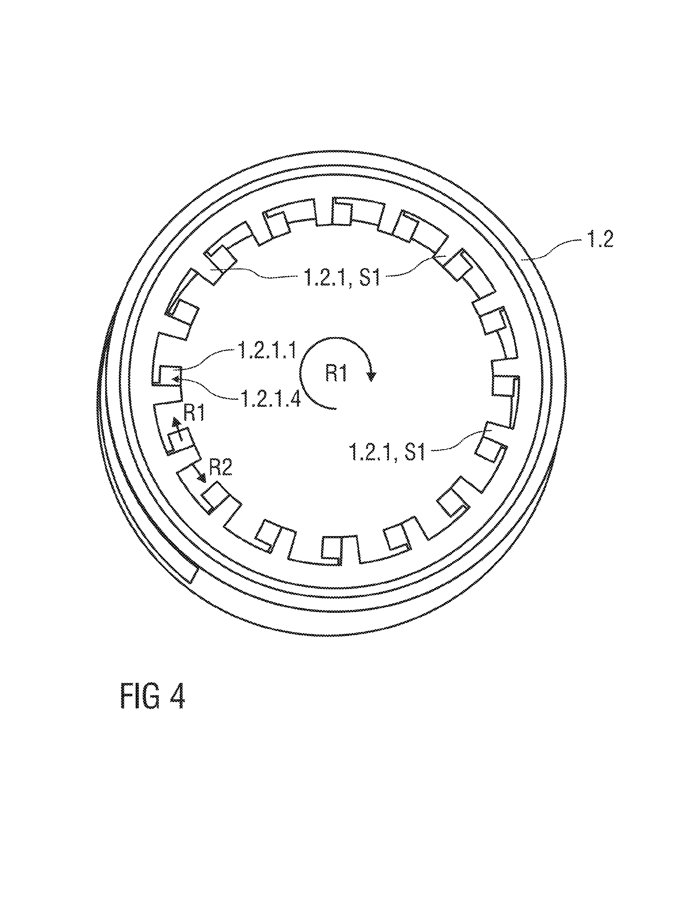

The first detent 1.2.1 is arranged to engage a corresponding second detent 2.2 of the medical cartridge 2 when the medical cartridge 2 is inserted into the cartridge holder 1. The first detents 1.2.1 are resiliently arranged and/or supported so as to allow for a deflection in a first rotational direction R1 best illustrated in figure 4. Deflection of the first detent 1.2.1 allows the corresponding second detent 2.2 to overcome the first detent 1.2.1 when a torque applied relative to the cartridge holder 1 and the medical cartridge 2 exceeds a maximal value.

Furthermore, the first set S1 of first detents 1.2.1 forms a bearing surface that is adapted to bear against the medical cartridge 2 to restrict an axial displacement parallel to the axis A in a distal direction D.

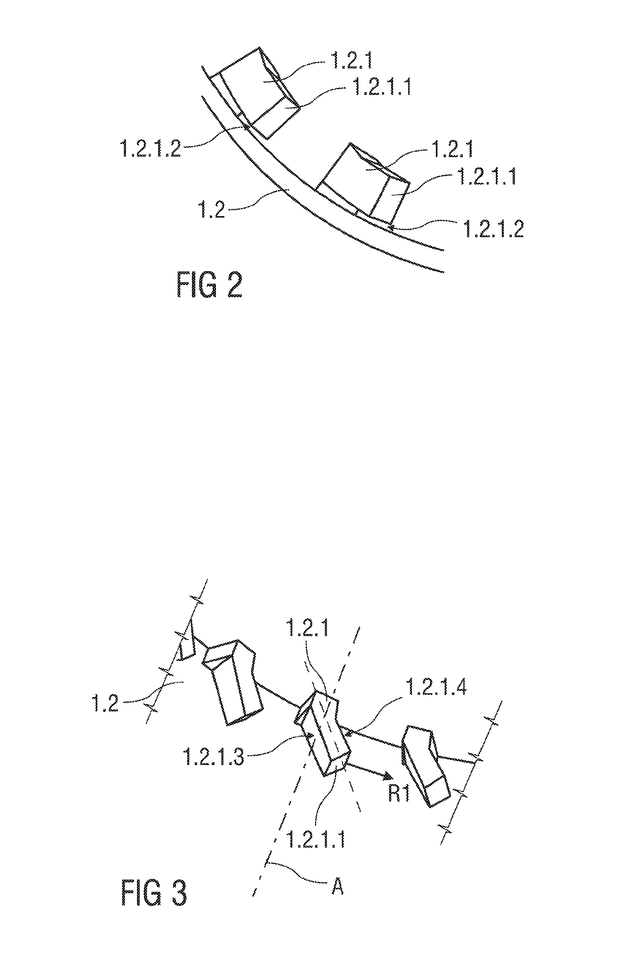

Figure 2 shows a section of the annular collar 1.2 formed to the distal end of the cartridge holder 1 and comprising the first set S1 of first detents 1.2.1. The first detent 1.2.1 comprises a free end 1.2.1.1 that is oriented towards a proximal direction P. The free end 1.2.1.1 is separated from the lateral wall of the annular collar 1.2 by an undercut 1.2.1.2 and is adapted to be deflected to provide a unidirectional torque limiter that restricts the torque that may occur between the cartridge holder 1 and the medical cartridge 2 to a maximal value to prevent the medical cartridge 2 from getting jammed within the cartridge holder 1.

Figure 3 shows another isometric view of the annular collar 1.2 located at the distal end of the cartridge holder 1 seen from a different angle. Each of the first detents 1.2.1 has a first surface 1.2.1.3 and a second surface 1.2.1.4 parallel to the first surface 1.2.1.3. The first and the second surfaces 1.2.1.3, 1.2.1.4 are inclined with respect to the axis A of the cartridge holder 1.

Figure 4 shows an isometric top view of the annular collar 1.2. The first detents 1.2.1 of the first set S1 are tilted towards the first rotational direction R1 and are resiliently arranged so as to allow for a deflection when the cartridge holder 1 is rotated with respect to the medical cartridge 2 in the first rotational direction R1. A rotation of the cartridge holder 1 with respect to the medical cartridge 2 in an opposition second rotational direction R2 is blocked. The first set S1 of first detents 1.2.1 therefore provides a unidirectional torque limiter that limits the torque applicable on the cartridge holder 1 in only the first rotational direction R1.

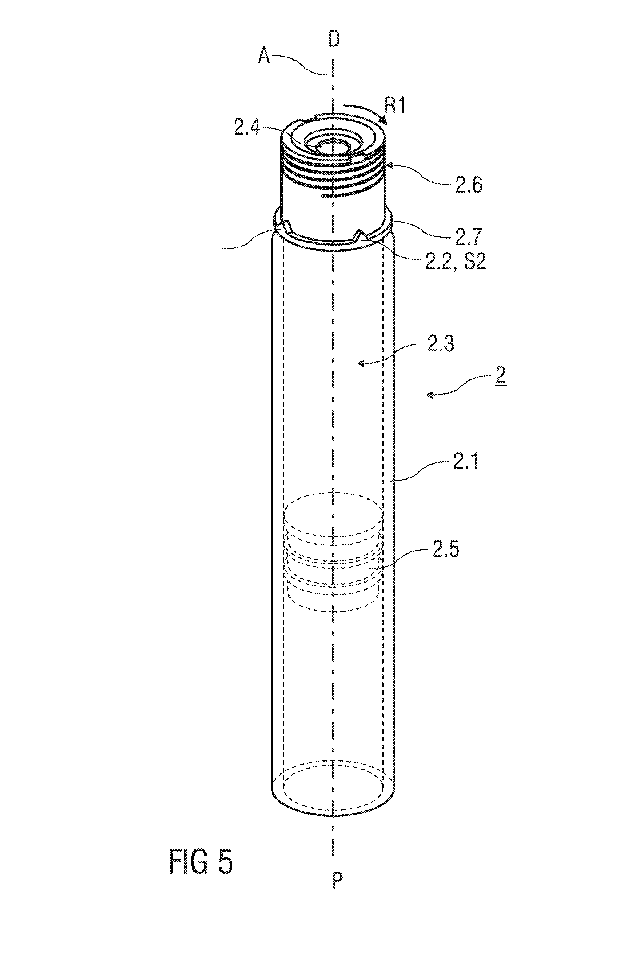

Figure 5 shows an isometric view of the substantially cylindrical medical cartridge 2 with the dose of the medicament contained within an inner cavity 2.3 of the barrel 2.1. A distal end of the inner cavity 2.3 is sealed by a septum 2.4, whereas a proximal end of the inner cavity 2.3 is sealed by a stopper 2.5.The stopper 2.5 is adapted to be engaged by a plunger (not illustrated) of the injection device to axially translate the stopper 2.5 in the distal direction D so as to dispose the dose of the medicament beneath the skin of the patient receiving the injection.

A screw thread 2.6 is formed into a distal end section of the medical cartridge 2. The screw thread is adapted to engage a correspondingly threaded element to attach the element to the medical cartridge 2. According to one possible embodiment of the invention, the element comprises a needle hub carrying a double-ended injection needle that is screwed onto the distal end section of the medical cartridge 2 after the medical cartridge 2 is inserted into the cartridge holder 1. The double-ended injection needle comprises a sharpened proximal and a sharpened distal end, wherein the distal end is adapted to be inserted into the skin of the patient receiving the injection. The proximal end of the injection needle punctures the septum 2.4 of the medical cartridge 2 when the needle hub is attached to the distal end section thereof to establish fluid communication of the injection needle with the inner cavity 2.3.

The orientation of the screw thread 2.6 corresponds to the first rotational direction R1 so as to allow the element and in particular the needle hub to be screwed onto the distal end section of the medical cartridge 2 by a rotation in the first rotational direction R1. Thus, the maximal torque that may be exerted upon the medical cartridge 2 retained within the cartridge holder 1 while the needle hub is attached is limited to the maximal value. In order to limit the torque in the first rotational direction, the second detent 2.2 is arranged to abut against the first surface 1.2.1.3 of the first detent 1.2.1 in the first radial direction. If the torque exerted upon the medical cartridge 2 in the first rotational direction R1 exceeds the maximal value, the first detent 1.2.1 that engages one of the corresponding second detents 2.2 are deflected so that the second detent 2.2 may overcome the first detent 1.2.1 and the medical cartridge 2 rotates about an angular segment with respect to the cartridge holder 1.

The second detent 2.2 formed to the distal end section of the cartridge 2 has a substantially triangular shape with at least one inclined side that corresponds to the first surface 1.2.1.3 of the first detent 1.2.1. A second set S2 of second detents 2.2 are arranged around a circumference of the distal end section of the medical cartridge 2. The second detent 2.2 projects in a radial outward direction to engage the first detent 1.2.1 of the cartridge holder 2. The second detents 2.2 of the second set S2 are equally spaced from each other around the circumference of the medical cartridge 2.

The number of second detents 2.2 in the second set S2 differs from the number of first detents 1.2.1 in the first set. In the embodiment illustrated in figures 1 to 6, the first set S1 comprises sixteen inwardly projecting first detents 1.2.1, whereas the second set S2 comprises only four outwardly projecting second detents 2.2. Thus, only a subset of first detents 1.2.1 of the first set S1 may be engaged by the second detents 2.2 of the second set S2 at any time. The subset of first detents 1.2.1 that are not engaged and thus not deflected are arranged to abut against an annular flange 2.7 projecting radial outwards from the distal end section of the medical cartridge 2 to limit an axial displacement of the medical cartridge 2 with respect to the cartridge holder 1 in the distal direction D.

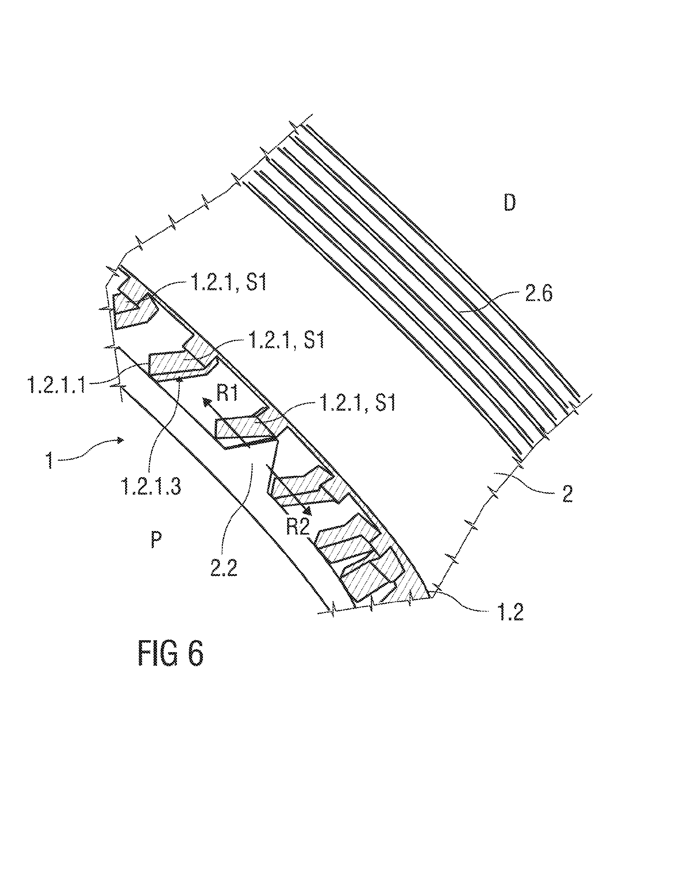

Figure 6 shows a section of the cartridge holder 1 with the medical cartridge 2 retained therein in an isometric view. The distal end section with the screw thread 2.6 of the medical cartridge 2 protrudes from a distal end of the cartridge holder 1 in the distal direction D. Rotation of the medical cartridge 2 with respect to the cartridge holder 1 in the first rotational direction R1 causes the triangular shaped second detent 2.2 to abut against the first surface 1.2.1.3 of the first detent 1.2.1. The first detent 1.2.1 is deflected in the first rotational direction R1 to make way for the second detent 2.2 if the applied torque exceeds the maximal value. Rotation of the medical cartridge 2 with respect to the cartridge holder 1 in the second rotational direction R2 causes the triangular shaped second detent 2.2 to abut against the free end 1.2.1.1 in a manner that blocks a further rotation of the medical cartridge 2 in the second rotational direction R2. Thus, the torque applied in the second rotational direction R2 is not limited. The second detent 2.2 may not overcome the first detent 1.2.1 in the second rotational direction R2 unless the assembly of the medical cartridge 2 and the cartridge holder is physically damaged, i.e. either the corresponding first or second detent 1.2.1, 2.2 breaks.

### List of References

- 1: cartridge holder
- 1.1: window
- 1.2: annular collar
- 1.2.1: first detent
- 1.2.1.1: free end
- 1.2.1.2: undercut
- 1.2.1.3: first surface
- 1.2.1.4: second surface
- 2: medical cartridge
- 2.1: barrel
- 2.2: second detent
- 2.3: inner cavity
- 2.4: septum
- 2.5: stopper
- 2.6: screw thread
- 2.7: annular flange
- P: proximal direction
- D: distal direction
- R1: first rotational direction
- R2: second rotational direction
- S1: first set
- S2: second set
- A: axis

## Claims

1. Cartridge holder (1) for an injection device capable of administering a dose of a medicament, wherein the cartridge holder (1) adapted to receive a medical cartridge (2) containing the dose of the medicament comprises an annular collar (1.2) with a first set (S1) of first detents (1.2.1) arranged around a circumference of the annular collar (1.2), wherein each first detent (1.2.1) projects from an inner surface of the annular collar (1.2) in a radial inward direction, the first detent (1.2.1) being adapted to engage a corresponding second detent (2.2) of the medical cartridge (2) to mount the medical cartridge (2) to the cartridge holder (1) and the first detent (1.2.1) being resiliently arranged and/or supported so as to provide a unidirectional torque limiter that restricts a torque between the cartridge holder (1) and the medical cartridge (2) in a first rotational direction (R1) to a maximal value.

2. Cartridge holder (1) according to claim 1,
**characterized in that** the first detents (1.2.1) of the first set (S1) arranged around the circumference of the annular collar (1.2) are regularly spaced apart from each other.

3. Cartridge holder according to claim 1 or 2,
**characterized in that** first detents (1.2.1) are adapted to engage the medical cartridge (2) so as to limit an axial displacement of the medical cartridge (2) with respect to the cartridge holder.

4. Cartridge holder (1) according to one of the previous claims,
**characterized in that** each of the first detents (1.2.1) has a first surface (1.2.1.3) and a second surface (1.2.1.4) parallel to the first surface (1.2.1.3), wherein the first surface (1.2.1.3) is inclined with respect to an axis (A) of the cartridge holder (1).

5. Cartridge holder (1) according to one of the previous claim,
**characterized in that** each of the first detents (1.2.1) comprises a free end (1.2.1.1) separated from the annular collar (1.2) by an undercut (1.2.1.2).

6. Cartridge holder (1) according to one of the previous claims,
**characterized in that** the annular collar (1.2) is integrally formed with the cartridge holder (1).

7. Cartridge holder (1) according to one of the previous claim,
**characterized in that** the annular collar (1.2) is arranged at a distal end of the cartridge holder (1).

8. Cartridge holder (1) according to one of the previous claims,
**characterized in that** an axial length of the cartridge holder (1) substantially corresponds to an axial dimension of the medical cartridge (2).

9. Assembly of a medical cartridge (2) and a cartridge holder (1) according to one previous claims, wherein the medical cartridge (2) is retained within the cartridge holder (1),
**characterized in that** the medical cartridge (2) comprises a second set (S2) of second detents (2.2) arranged around a circumference of the medical cartridge (2), wherein the second detents (2.2) project in a radial outward direction to engage the first detents (1.2.1) and at least one second detent (2.2) engages at least one first detent (1.2.1) to provide the unidirectional torque limiter.

10. Assembly according to claim 9,
**characterized in that** the second detent (2.2) comprises a triangular shape with an inclined side arranged to abut on second surface (1.2.1.4) of the first detent (1.2.1) to restrict the torque between the cartridge holder (1) and the medical cartridge (2) in the first rotational direction (R1) to the maximal value.

11. Assembly according to claim 9 or 10,
**characterized in that** the second detents (2.2) of the second set (S2) arranged around the circumference of the medical cartridge (2) are regularly spaced apart from each other.

12. Assembly according to claim 9 to 11,
**characterized in that** the medical cartridge (2) comprises an annular flange (2.7) arranged to abut against at least one of the first detents (1.2.1) to limit an axial translation of the medical cartridge (2) with respect to the cartridge holder (1).

13. Assembly according to one of the claims 9 to 12,
**characterized in that** a screw thread (2.6) adapted to be connected to a correspondingly threaded element, wherein an orientation of rotation of the screw thread (2.6) corresponds to the first rotational direction (R1) limited by the torque limiter of the cartridge holder (1).

14. Assembly according to claim 12,
**characterized in that** the screw thread (2.6) is arranged at a distal end section of the medical cartridge (2) and protrudes from the cartridge holder (1) in a distal direction (D).

15. Assembly according to claim 13 or 14,
**characterized in that** the element comprises a needle hub with an injection needle attached thereto.
